# EUROPEAN PATENT APPLICATION

(11) **EP 4 404 094 A1**
(43) Date of publication of application: **24.07.2024**
(21) Application number: 22869850.2
(22) Date of filing: 06.09.2022
(51) Int. Cl.: G06F 21/60, G06K 19/06

(54) **ENCRYPTING APPARATUS AND ENCRYPTING METHOD**

(30) Priority: 17.09.2021 JP 2021151766
(71) Applicant: A.T COMMUNICATIONS CO., LTD., Tokyo 110-0014 (JP)
(72) Inventor: TOYOIZUMI Hiroshi, Tokyo 110-0014 (JP); AZUMA Youichi, Tokyo 110-0014 (JP); MORIMOTO Hajime, Tokyo 104-0033 (JP); MORIMOTO Atsushi, Tokyo 104-0033 (JP)
(74) Representative: Peters, Sebastian Martinus
(86) International application number: PCT/JP2022/033364
(87) International publication number: WO 2023/042703

(57) **Abstract**

[Problem] To provide a digital data encrypting method with which data concealment is enhanced.

[Solution] A data encrypting method using an encrypting apparatus, the method including a first partial data input step in which first partial data that is a portion of data is inputted to the encrypting apparatus, a first encryption step in which the first partial data is encrypted and first encrypted data is obtained, a first partial data erasure step in which the first partial data is erased from the encrypting apparatus, a second partial data input step in which second partial data that is partial data of the aforementioned data other than the first partial data is inputted to the encrypting apparatus, and a second encryption step in which the second partial data is encrypted and second encrypted data is obtained.

## Description

### TECHNICAL FIELD

The present invention relates to a digital data encrypting apparatus and encrypting method.

### BACKGROUND ART

JP 2021-101629 A describes a genome analysis device and a gene analysis device. By using such a genome analysis device and gene analysis device, human genome information and gene information can be analyzed. However, it is desirable to keep human genome information and gene information confidential.

Japanese Patent No. 6664785 describes a data restoration device. The invention described in this publication separates data into two parts. One of part of the data is saved via a network, the remaining part of the data is saved locally, and then the data is restored. The data restoration device is able to reduce the data stored locally.

By dividing and storing data as described above, data confidentiality can be enhanced. On the other hand, when data is encrypted and divided, the encrypted data remains temporarily in the encrypting apparatus, so if this data is stolen, there is a risk that the data will be stolen by a third party.

### CITATION LIST

### PATENT LITERATURE

Patent Literature 1: JP 2021-101629 A
Patent Literature 2: Japanese Patent No. 6664785

### SUMMARY OF INVENTION

### TECHNICAL PROBLEM

An object as described in the present disclosure is to provide a digital data encrypting apparatus and encrypting method that enhances data confidentiality.

### SOLUTION TO PROBLEM

The object described above is based on knowledge that it is possible to receive a part of data, encrypt and then delete the received data, receive and encrypt the remaining part of the data (remaining part), such that confidentiality of data can be enhanced without the entire data existing in the apparatus.

A first description relates to an encrypting method of encrypting data using an encrypting apparatus.

This method includes a first partial data input step (S101), a first encryption step (S102), a first partial data erasure step (S103), a second partial data input step (Sill), and a second encryption step (S112).

The first partial data input step (S101) is a step in which first partial data, which is a part of data, is input into the encrypting apparatus.

The first encryption step (S102) is a step of encrypting the first partial data input to the encrypting apparatus in the first partial data input step (S101) to obtain first encrypted data.

The first partial data erasure step (S103) is a step of erasing the first partial data from the encrypting apparatus.

The second partial data input step (Sill) is a step in which, after the first partial data erasure step (S103), second partial data, which is partial data of the data other than the first partial data, is input to the encrypting apparatus.

The second encryption step (S112) is a step of encrypting the second partial data input to the encrypting apparatus in the second partial data input step to obtain second encrypted data.

In the method described above, the entire original data does not exist in the encrypting apparatus even temporarily. Therefore, this encrypting apparatus can be said to be an encrypting apparatus having a high degree of confidentiality.

Preferably, the above-described method further includes a first encrypted data transmission step (S104) and a first encrypted data erasure step (S105) before the second partial data input step (5111).

The first encrypted data transmission step (S104) is a step of transmitting the first encrypted data from the encrypting apparatus to a first data storage terminal.

The first encrypted data erasure step (S105) is a step of erasing the first encrypted data from the encrypting apparatus after the first encrypted data transmission step (S104).

In this method, there is no moment when encrypted data in which the entire original data has been encrypted exists in the encrypting apparatus. Therefore, this method can achieve an extremely high degree of confidentiality. This partial data may be restored using a technique such as that disclosed in Japanese Patent No. 6664785, for example.

Preferably, the above-described method further includes a second encrypted data transmission step (S114). The second encrypted data transmission step (S114) is a step of transmitting the second encrypted data from the encrypting apparatus to the second data storage terminal. The first data storage terminal and the second data storage terminal are terminals having physically different storage devices.

Examples of the above-described data include gene analysis result information, diagnostic image data, and measurement data.

Preferably the method described above is a method that, together with being able to further keep encrypted data confidential by dividing the encrypted data, is a method that, even in a case where information necessary for restoration is lost, as long as personal information or the like can be identified, is able to recover the necessary information. This method further includes: a step of dividing the first encrypted data to obtain first data and second data, a step of storing the first data in a first data storage portion; a step of converting the second data into code information to obtain encoded second data; a step of outputting the encoded second data as outputted encoded second data; a step of correlating and storing the encoded second data and data identification information regarding the encoded second data in an encoded second data storage portion; a step of reading the encoded second data from the encoded second data storage portion when the data identification information has been input to obtain the outputted encoded second data; a step of reading the encoded second data based on the outputted encoded second data; and a step of reading the first data from the first data storage portion based on the encoded second data and restoring the original data. In the above example, only the first encrypted data is divided. However, all of n pieces of encrypted data may be divided and encrypted, or any one or more of n pieces of encrypted data may be divided and encrypted.

This disclosure next describes an encrypting apparatus. This encrypting apparatus is an apparatus for implementing the above-described method. Such an encrypting apparatus 1 includes a data input portion 11, a first encrypting portion 13, a first partial data erasure portion 15, an input control portion 17, and a second encrypting portion 21.

The data input portion 11 is an element into which data is input.

The first encrypting portion 13 is an element that, when first partial data as a part of data is input to the data input portion, encrypts the first partial data to obtain first encrypted data.

The first partial data erasure portion 15 is an element that erases the first partial data after the first encrypted data is obtained.

The input control portion 17 is an element that performs control such that after the first partial data has been erased, the data input portion inputs second partial data that is partial data other than the first partial data of the data.

The second encrypting portion 21 is an element that encrypts the second partial data to obtain second encrypted data when the second partial data is input to the data input portion.

Preferably, this apparatus further includes a data management system (data division and encryption processing portion). The encrypting apparatus further includes: a data division portion that divides the first encrypted data to obtain first data and second data; a first data storage portion that stores the first data; a code conversion portion that converts the second data into code information to obtain encoded second data; an encoded second data output portion that outputs the encoded second data as outputted encoded second data; a code information reading portion that reads the encoded second data based on the outputted encoded second data; a data restoration portion that, based on the encoded second data read by the code information reading portion, reads the first data from the first data storage portion and restores the original data; an encoded second data storage portion that correlates and stores the encoded second data and data identification information regarding the encoded second data; and a code output portion that, when the data identification information is input, reads the encoded second data from the encoded second data storage portion and obtains the outputted encoded second data.

### ADVANTAGEOUS EFFECTS OF INVENTION

According to this disclosure, it is possible to provide a digital data encrypting apparatus and encrypting method that enhance data confidentiality.

### BRIEF DESCRIPTION OF DRAWINGS

FIG. 1 is a flowchart for explaining an example of an encrypting method.
FIG. 2 is a block diagram for explaining an encrypting apparatus.
FIG. 3 is a conceptual diagram for explaining a modification of a first encryption step.

### DESCRIPTION OF EMBODIMENTS

Hereinafter, embodiments for implementing the present invention will be described using the drawings. The present invention is not limited to the embodiments described below, and also includes modifications made from the following embodiments as appropriate within a range obvious to a person skilled in the art.

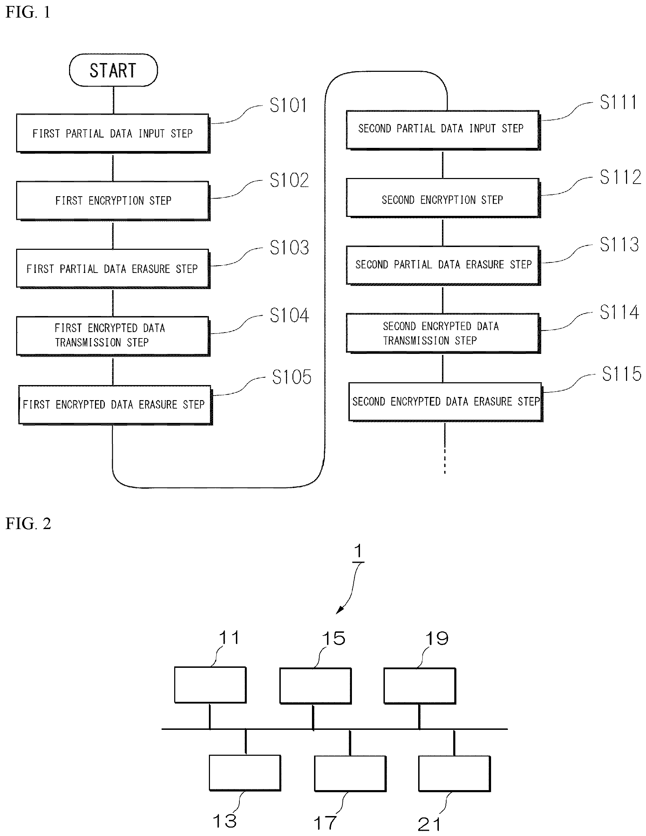

FIG. 1 is a flowchart for explaining an example of an encrypting method. In this example, the steps include a first partial data input step (S101), a first encryption step (S102), a first partial data erasure step (S103), a first encrypted data transmission step (S104), a first encrypted data erasure step (S105), a second partial data input step (Sill), a second encryption step (S112), a second partial data erasure step (S113), a second encrypted data transmission step (S114), and a second encrypted data erasure step (S115). Encryption and confidentiality can also be achieved through the above partial steps.

FIG. 2 is a block diagram for explaining the encrypting apparatus. This encrypting apparatus includes a computer, and each element described below is an element implemented by the computer. The computer may be any of various types of terminals, mobile terminals, notebook computers, personal computers, servers, and computer-server systems. In addition, this system may be implemented by a processor, or by cooperation between various types of hardware and software such as a program.

As illustrated in FIG. 2, an encrypting apparatus 1 includes a data input portion 11, a first encrypting portion 13, a first partial data erasure portion 15, an input control portion 17, and a second encrypting portion 21. As illustrated in FIG. 2, the encrypting apparatus 1 may further include a data transmission portion 19. Note that the first encrypting portion 13 and the second encrypting portion 21 may be physically implemented using the same element. In addition to the encrypting apparatus 1, the encryption system may include other terminals and servers that can exchange information with the encrypting apparatus 1. Preferably, the encrypted data is stored in those terminals and servers. Moreover, it is preferable that the encrypted data be distributed and stored among a plurality of terminals and servers. The encrypting apparatus 1 is a device that implements the encrypting method described in the present disclosure. The technique according to the present disclosure also includes a program for implementing the encrypting method on a computer or a processor, and a computer-readable information recording medium (DVD, Blu-ray disc, CD, floppy disk, USB, and the like) on which the program is recorded.

The computer has an input portion, an output portion, a control portion, a calculation portion, and a storage portion, and each element is connected by a bus or the like so that information can be exchanged. For example, the storage portion may store programs or various types of information. The computer may include a processor and perform various types of calculations and processes based on instructions from a program. In a case where predetermined information is input from the input portion, the control portion reads a control program stored in the storage portion. The control portion reads information stored in the storage portion as appropriate and transmits the information to the calculation portion. In addition, the control portion transmits appropriately input information to the calculation portion. The calculation portion performs arithmetic processing using the various types of received information and stores the result in the storage portion. The control portion reads the calculation result stored in the storage portion and outputs the calculation result from the output portion. Various types of processes are executed in this way. Each element described below may correspond to any element of a computer. An example of the output portion is a monitor or a screen.

The method will be explained using gene analysis as an example. For example, using a PCR device, a base sequence of a certain gene can be obtained in sequence. In a preferred example, before full-length data of a certain gene (entire data to be encoded) is analyzed, the data is transmitted from the analysis device to the encrypting apparatus.

The first partial data input step (S101) is a step in which first partial data, which is a part of data, is input into the encrypting apparatus. Partial data of data to be encrypted is transmitted from a data source such as the analysis device described above or a measurement device to the encrypting apparatus. For example, the data input portion 11 of the encrypting apparatus 1 receives the partial data. Then, the partial data is input to the encrypting apparatus. The input portion of the computer functions as the data input portion 11. The encrypting apparatus 1 is a device for encrypting data so that it cannot be displayed or decoded as it is. Examples of encrypting methods will be described later. The partial data may be stored in the storage portion as appropriate.

Another example of data that is different from that described above is diagnostic image data. For example, an MRI diagnostic image consists of a plurality of sliced images of a target region, resulting in a series of diagnostic image data. Any one of the plurality of image data may be the first partial data. Moreover, any one of a plurality of image data or a part of any one image data such as an X-ray photograph (for example, one piece of image data divided into multiple pieces of image data based on pixel density, and one having a certain pixel density) may be the first partial data. The same applies to the second partial data and subsequent data. In this way, the first partial data itself is data whose meaning is difficult to understand, and thus confidentiality becomes extremely high.

An example of the above-described data is measurement data. Examples of measurement data include data such as air temperature, temperature, humidity, strain, and light intensity that are measured using a measuring device. The measurement data is preferably measurement data that changes over time.

The first encryption step (S102) is a step that encrypts the first partial data (ABC) input to the encrypting apparatus in the first partial data input step (S101) to obtain first encrypted data (abc). For example, the first encrypting portion 13 encrypts the first partial data to obtain the first encrypted data. The control portion of the computer may read a program from the storage portion, read various types of information from the storage portion based on the instructions from the program, and cause the calculation portion to perform calculations on the first partial data to obtain the first encrypted data. The obtained first encrypted data may then be appropriately stored in the storage portion. Encryption and encoding methods are publicly known. This encryption step can use any known encrypting method as appropriate.

The first partial data erasure step (S103) is a step of erasing the first partial data (ABC) from the encrypting apparatus. The first partial data erasure portion 15 erases the first partial data (ABC) stored in the storage portion. For example, the control portion of the encrypting apparatus erases the first partial data (ABC) stored in the storage portion according to instructions from a program. As a result, the first partial data no longer exists in the encrypting apparatus. However, the first encrypted data (abc) may be stored in the storage portion of the encrypting apparatus.

A preferred method includes a first encrypted data transmission step (S104). The first encrypted data transmission step (S104) is a step of transmitting the first encrypted data (abc) from the encrypting apparatus to a first data storage terminal. The control portion of the encrypting apparatus receives instructions from a program and transmits the first encrypted data (abc) from the output portion (first encrypted data transmission portion) to a first data storage terminal. As a result, the first data storage terminal receives the first encrypted data (abc) and stores the data in a storage portion of the first data storage terminal as appropriate. Note that in this encrypted data transmission step, the encrypted data may be transmitted to a plurality of data storage terminals. In particular, in the first encryption step (S102), in a case where the encrypted data is divided into a plurality of types of data, the divided encrypted data may be transmitted to a plurality of data storage terminals.

A preferred method includes a first encrypted data erasure step (S105). The first encrypted data erasure step (S105) is a step of deleting the first encrypted data (abc) from the encrypting apparatus after the first encrypted data transmission step (S104). The first encrypted data erasure portion of the encrypting apparatus erases the first encrypted data (abc) from the storage unit. The control portion of the encrypting apparatus receives a program instruction and erases the first encrypted data (abc) from the storage portion. As a result, the first encrypted data (abc) no longer exists in the encrypting apparatus 1.

The second partial data input step (Sill) is a step in which second partial data, which is partial data of the data other than the first partial data, is input into the encrypting apparatus. For example, the control portion of the encrypting apparatus, based on program instructions, controls an input device so as not to receive information input from an external device until any one of the above-described first partial data erasure step (S103), the above-mentioned first encrypted data transmission step (S104), or the first encrypted data erasure step (S105) is completed. The control portion and program function as the input control portion 17. As a result, in a state where the first partial data (ABC) (and the first encrypted data (abc)) does not exist in the encrypting apparatus, the second partial data (DEF), which is partial data of the data other than the first partial data, is input. In this step, the same processing as in the first partial data input step (S101) may be performed.

The second encryption step (S112) is a step of encrypting the second partial data input to the encrypting apparatus in the second partial data input step to obtain second encrypted data (def). This step may be performed in the same manner as the first encryption step (S102).

The second partial data erasure step (S113) is a step of erasing the second partial data (DEF) from the encrypting apparatus. The second partial data erasure step (S113) may be performed in the same manner as the first partial data erasure step (S103).

The second encrypted data transmission step (S114) is a step of transmitting the second encrypted data from the encrypting apparatus to the second data storage terminal. The first data storage terminal and the second data storage terminal are terminals having physically different storage devices. The control portion of the encrypting apparatus receives instructions from a program and transmits the second encrypted data (def) from the output portion (second encrypted data transmission portion) to a second data storage terminal. As a result, the second data storage terminal receives the second encrypted data (def) and stores the data in a storage portion of the second data storage terminal as appropriate. The storage device that stores the first encrypted data (abc) of the first data storage terminal and the storage device that stores the second encrypted data (def) of the second data storage terminal are different (the first data storage terminal and the second data storage terminal may be physically the same). As a result, the first encrypted data (abc) and the second encrypted data (def) do not exist in one storage device, and thus even in the encrypted state, it is possible to prevent information regarding the first partial data and the second partial data from being collected in one storage device. In this way, the method, the device, and the system can enhance data confidentiality.

The second encrypted data erasure step (S115) is a step of erasing the second encrypted data (def) from the encrypting apparatus after the second encrypted data transmission step (S114). The second encrypted data erasure step (S115) may be performed in the same manner as the first encrypted data erasure step (S105).

Thereafter, the above-described steps may be further repeated depending on the number of divided data. However, when the data is divided into n pieces, the control portion of the encrypting apparatus may transmit nth encrypted data to an nth storage terminal, or may transmit the data to the first or second storage terminal, according to instructions from the program. In any case, it is possible to prevent all encrypted data from being collected in one storage terminal.

By appropriately collecting and decrypting the distributed encrypted data, the original data can be restored.

Next, a modification of the first encryption step (S102) will be described. This modification is implemented by a data management system. Preferably, this data management system is part of the encrypting apparatus described above. This system includes a data division portion, a first data storage portion, a code conversion portion, an encoded second data output portion, a code information reading portion, and a data restoration portion. Preferably, this data management system further includes an encoded second data storage portion and a code output portion.

The data division portion is an element for obtaining first data and second data obtained by dividing original data (nth partial data or nth encrypted data). The first data storage portion is an element for storing first data. The code conversion portion is an element for converting the second data into code information to obtain encoded second data. The encoded second data output portion is an element for outputting the encoded second data as outputted encoded second data. The code information reading portion is an element for reading encoded second data based on the outputted encoded second data. The data restoration portion is an element for reading the first data from the first data storage portion and restoring the original data based on the encoded second data. The encoded second data storage portion is an element for correlating and storing encoded second data and data identification information for the encoded second data. The code output portion is an element for reading encoded second data from the encoded second data storage portion and obtaining outputted encoded second data in a case where data identification information is input.

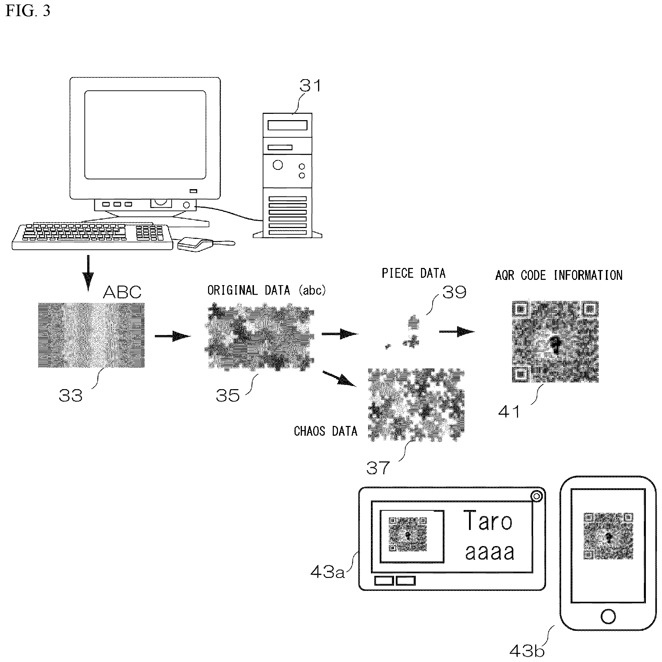

FIG. 3 is a conceptual diagram for explaining a modification of the first encryption step. In this example, first partial data (ABC) 33 or first encrypted data (abc) 35 is divided. In the example of FIG. 3, the first encrypted data is divided. Data obtained from a measurement device such as the gene analysis device 31 is sequentially input to the system. For example, in a case where the length of the target gene is 100 mer, amino acid residues are obtained one by one starting from an N-terminal side. Before the full-length gene of the target gene is analyzed, the obtained amino acid residue information is input to the system. In the example of FIG. 3, partial data of a full-length gene is input to the system, and the encrypted data of the partial data is divided. The data division portion of the encrypting apparatus divides the first partial data (ABC) or the first encrypted data (abc), which is the original data, to obtain first data and second data. The control portion and the calculation portion of the computer function as the data division portion. The first data is also called chaos data 37 and the second data is also called piece data 39. It is preferable that the first partial data (ABC) is not able to be restored using only chaos data or piece data.

The piece data is output as code information, and thus it is preferable that the amount of first data (chaos data) 37 be greater than the amount of the second data (piece data) 39. A data amount ratio is a ratio of the data amount of the first data (chaos data)/the data amount of the second data (piece data) . In this case, the data amount ratio is preferably 3 or more and 10⁴ or less, 3 or more and 10³ or less, 3 or more and 10² or less, 4 or more and 10² or less, or 10 or more and 10⁴ or less.

The storage portion of the encrypting apparatus stores first data (for example, chaos data) 37. The storage portion may exist on an online server or may exist offline. In this way, the first data (chaos data) may be stored in the storage portion of a local server. Moreover, the first data may be stored on various types of servers via the Internet. Furthermore, in a case where the first partial data (ABC) cannot be restored using only the first data, the first data may be made public. In that case, the first data may be stored in association with an identifier associated with the second data. The first data may also be stored in the local server and an emergency server that functions in place of the local server when the local server fails. In addition, the first data may be stored in a storage portion of a local server and a standalone storage unit (for example, a standalone computer or a standalone hard disk that is not normally stored on a network). By doing so, the first data can be used even in an emergency.

The second data (piece data) 39 may be stored in the storage portion of the encrypting apparatus or a specific local server, or may be stored in various types of servers via the Internet. However, the second data is preferably stored in a storage portion of a specific computer or a storage unit of a local server that is connected to the encrypting apparatus via an intranet or the like, and placed in an environment where it cannot be accessed by a third party. A storage portion of the computer functions as a second data storage portion.

However, it is preferable that the second data 39 exist only in a cache and that an encoding process be performed without the second data 39 being stored on a hard disk or the like. In addition, after the second data is encoded, the second data may be erased from the storage portion. In this way, the second data no longer exists, and thus data confidentiality can be enhanced.

An encrypting portion converts the second data (piece data) 39 into code information to obtain encoded second data 41. The encoded second data is information for outputting code information. The computer can obtain encoded second data by the control unit causing the calculation portion to perform appropriate calculations on the second data based on program instructions. The encoded second data obtained in this manner is appropriately stored in the storage portion. Methods and apparatus for encoding data are known. For example, Japanese Patent No. 6664785 describes a data code generation server. In addition, Japanese Patent No. 5945376 describes a two-dimensional code generation device. By using such a device, data can be encoded. An example of the encoded second data is AQR code information (registered trademark) for printing a two-dimensional code or color QR code (registered trademark). The encoded second data may be stored in the storage portion. A color two-dimensional code or a color QR code (registered trademark) means a two-dimensional code that has colors as well as black and white. The colored portion may be, for example, a personal photo or a personal avatar. In addition, this colored portion may be a specific character. When the outputted encoded second data has such a colored portion, a user becomes attached to the outputted encoded second data.

The output portion of the encrypting apparatus outputs the encoded second data as outputted encoded second data 43a, 43b. For example, the control portion, the calculation portion, and the output portion (printer, interface, and communication portion) of the computer function as the encoded second data output portion. Preferably, the output portion includes a color printer. The outputted encoded second data may be identification information from which the encoded second data stored in the storage portion can be read. In this case, when the outputted encoded second data is input, the computer can read the encoded second data from the storage portion using the outputted encoded second data.

An example of the outputted encoded second data is a color two-dimensional code (AQR code (registered trademark)) 43a printed on a medium. Another example of outputted encoded second data is code information printed on a medium that is recognized by ultraviolet or infrared light. In this case, the code information reading portion reads the encoded second data based on code information recognized by ultraviolet or infrared light. Preferred examples of outputted encoded second data include visible code information (two-dimensional code, QR code (registered trademark)) as well as code information recognized by ultraviolet or infrared light. In this case, for example, even if someone else copies the medium and copies the visible code information portion, the code information recognized by ultraviolet or infrared light cannot be restored, so the encoded second data cannot be completely copied after being output, and unauthorized use can be prevented. Another example of the outputted encoded second data is a QR code (registered trademark) 43b sent to a personal terminal (for example, a mobile terminal). In this case, a terminal related to an individual, identification information (e-mail address) related to the individual, or the like is registered, and the outputted encoded second data may be transmitted using the registered identification information. It is preferable that the computer, based on the outputted encoded second data, store the encoded second data and the second data in a storage portion so as to be restorable. In this way, even in a case where the distributed outputted encoded second data is lost, it is possible to reproduce the encoded second data using data identification information such as personal information.

Next, an example of decoding will be explained.

A decoding device reads encoded second data based on the outputted encoded second data. For example, Japanese Patent No. 6664785 and Japanese Patent No. 5945376 describe devices for reading two-dimensional codes. For example, the outputted encoded second data (color QR code (registered trademark) printed on a medium or color QR code (registered trademark) displayed on a mobile device) is held over an imaging portion. As a result, the imaging portion obtains an image of the outputted encoded second data and inputs the obtained image to the system. The control portion causes the calculation portion to analyze the image based on a program instruction, and reads the encoded second data from the storage portion based on the outputted encoded second data.

The decoding device reads the first data from the data storage portion based on the encoded second data and restores the original data. In this step, the encoded second data is used to read the second data from the storage portion. On the other hand, the first data is, for example, stored in the storage portion in association with the second data. The first data is read using the read second data. Thereafter, the first partial data is restored using the first data and the second data.

Data may be similarly divided in the second encryption step (S112) and subsequent encryption steps. The plurality of second data may be collected into one encoded second data.

### INDUSTRIAL APPLICABILITY

This invention can be used in information and communications related industries.

### REFERENCE SIGNS LIST

- 1: encrypting apparatus
- 11: Data input portion
- 13: First encrypting portion
- 15: First partial data erasure portion
- 17: Input control portion
- 19: Data transmission portion
- 21: Second encrypting portion

## Claims

1. A data encrypting method using an encrypting apparatus, comprising:
a first partial data input step in which first partial data as a part of the data is input to the encrypting apparatus;
a first encryption step of encrypting the first partial data input to the encrypting apparatus in the first partial data input step to obtain first encrypted data;
a first partial data erasure step of deleting the first partial data from the encrypting apparatus;
a second partial data input step in which second partial data of the data is input to the encrypting apparatus after the first partial data erasure step, the second partial data being partial data other than the first partial data; and
a second encryption step of encrypting the second partial data input to the encrypting apparatus in the second partial data input step to obtain second encrypted data.

2. The method according to claim 1, further comprising: a first encrypted data transmission step of transmitting the first encrypted data from the encrypting apparatus to a first data storage terminal before the second partial data input step; and a first encrypted data erasure step of deleting the first encrypted data from the encrypting apparatus after the first encrypted data transmission step.

3. The method according to claim 2, further comprising a second encrypted data transmission step of transmitting the second encrypted data from the encrypting apparatus to a second data storage terminal; wherein the first data storage terminal and the second data storage terminal are terminals having physically different storage devices.

4. The method according to claim 1, wherein the data is gene analysis result information.

5. The method according to claim 1, wherein the data is diagnostic image data.

6. The method according to claim 1, wherein the data is measurement data.

7. The method according to claim 1, further comprising:
a step of dividing the first encrypted data to obtain first data and second data;
a step of storing the first data in a first data storage portion;
a step of converting the second data into code information to obtain encoded second data;
a step of outputting the encoded second data as outputted encoded second data;
a step of correlating and storing the encoded second data and data identification information regarding the encoded second data in an encoded second data storage portion;
a step of, in a case where the data identification information is input, reading the encoded second data from the encoded second data storage portion to obtain the outputted encoded second data;
a step of reading the encoded second data based on the outputted encoded second data; and
a step of, based on the encoded second data, reading the first data from the first data storage portion and restoring the original data.

8. An encrypting apparatus, comprising:
a data input portion to which data is input;
a first encrypting portion configured to encrypt first partial data to obtain first encrypted data when first partial data as a part of the data is input to the data input portion;
a first partial data erasure portion configured to erase the first partial data after the first encrypted data is obtained by the first encrypting portion;
an input control portion configured to perform control such that after the first partial data has been erased, the data input portion inputs second partial data as partial data other than the first partial data of the data; and
a second encrypting portion configured to encrypt the second partial data to obtain second encrypted data when the second partial data is input to the data input portion.

9. The encrypting apparatus according to claim 8, further comprising:
a data division portion configured to divide the first encrypted data to obtain first data and second data;
a first data storage portion configured to store the first data;
a code conversion portion configured to convert the second data into code information to obtain encoded second data;
an encoded second data output portion configured to output the encoded second data as outputted encoded second data;
a code information reading portion configured to read the encoded second data based on the outputted encoded second data;
a data restoration portion configured to, based on the encoded second data read by the code information reading portion, read the first data from the first data storage portion and restore the original data;
an encoded second data storage portion configured to correlate and store the encoded second data and data identification information regarding the encoded second data; and
a code output portion configured to, when the data identification information is input, read the encoded second data from the encoded second data storage portion and obtain the outputted encoded second data.
